# EUROPEAN PATENT SPECIFICATION

(11) **EP 4 397 647 B1**
(45) Date of publication and mention of the grant of the patent: **29.10.2025**
(21) Application number: 23863411.7
(22) Date of filing: 01.09.2023
(51) Int. Cl.: C07C 29/14, C07C 29/74, C07C 31/20, C07C 29/141, C07C 45/75, C07C 45/80, C07C 45/82

(54) **METHOD FOR PREPARING NEOPENTYL GLYCOL**
VERFAHREN ZUR HERSTELLUNG VON NEOPENTYLGLYKOL
PROCÉDÉ DE PRÉPARATION DE NÉOPENTYLGLYCOL

(30) Priority: 08.09.2022 KR 20220114479; 25.08.2023 KR 20230111947
(43) Date of publication of application: 10.07.2024
(73) Proprietor: LG Chem, Ltd., Yeongdeungpo-gu Seoul 07336 (KR)
(72) Inventor: KIM, Sung Kyun, Daejeon 34122 (KR); LEE, Sung Kyu, Daejeon 34122 (KR); BAEK, Gi Su, Daejeon 34122 (KR); HA, Na Ro, Daejeon 34122 (KR)
(74) Representative: Goddar, Heinz J.
(86) International application number: PCT/KR2023/013046
(87) International publication number: WO 2024/053935

(56) References cited:
- EP-A1- 3 219 698
- WO-A1-2016/022601
- JP-A- H09 110 792
- KR-A- 20170 029 311
- KR-B1- 100 852 283
- US-A- 4 935 555

## Description

### [Technical Field]

The present invention relates to a method for preparing neopentyl glycol.

### [Background Art]

Neopentyl glycol may be generally prepared by performing an aldol condensation reaction of isobutyl aldehyde and formaldehyde in the presence of a catalyst to form hydroxypivaldehyde and proceeding with a hydrogenation reaction of the hydroxypivaldehyde.

However, formic acid is produced from a Cannizzaro side reaction occurring during an aldol condensation reaction process, the catalyst is changed into a catalyst salt by the formic acid, and the catalyst salt is in the form of an aqueous phase and conventionally treated as waste water. In addition, hydroxypivalic acid-neopentyl glycol ester (HPNE) produced by a Tishchenko reaction which is another side reaction of the aldol condensation reaction process is completely discarded with high-boiling point by-products during a process of purifying neopentyl glycol. However, since the hydroxypivalic acid-neopentyl glycol ester (HPNE) is a high value-added product in itself, it needs to be recovered and used.

That is, an environmental pollution problem is caused by the discarded catalyst salt and HPNE, and as an added catalyst is produced as a catalyst salt and discarded, production costs increase during a process of continuously adding a new catalyst.

Therefore, a process which is environmentally friendly and may reduce energy costs by recovering the discarded catalyst and HPNE as such should be introduced.

EP 3 219 698 A1 discloses a method of preparing glycol by means of an apparatus comprising an aldol reactor, an extractor for extracting an aldol product, unsaturated aldehyde, using an organic solvent that is not mixed with water; a distillation column for removing a raw material from a solution extract that is discharged from the extractor; a hydrogenation reactor for hydrogenating a solution extract that is discharged from the distillation column; and a divided-wall distillation column for isolating glycol from a hydrogenated solution product that is discharged from the hydrogenation reactor, wherein the hydrogenation reactor is a fixed-bed catalytic reactor that is filled with a copper-based catalyst.

### [Disclosure]

### [Technical Problem]

In order to solve the problems mentioned in the Background Art, an object of the present invention is to provide a method for preparing neopentyl glycol, which is environmentally friendly in the entire process and may further reduce energy costs while obtaining high-purity neopentyl glycol with a high recovery rate.

### [Technical Solution]

In one general aspect, provided is a method for preparing neopentyl glycol that includes: performing an aldol condensation reaction of a formaldehyde aqueous solution and isobutyl aldehyde in the presence of a catalyst in an aldol reactor to obtain a first reaction product including hydroxypivaldehyde; supplying the first reaction product to an aldol extraction column and bringing the first reaction product into contact with an extractant to obtain an extract including hydroxypivaldehyde and a raffinate including a catalyst salt; supplying the raffinate to a saponification reactor to reduce the catalyst salt to a catalyst; supplying the discharge stream from the saponification reactor to a catalyst recovery column to separate a stream including the catalyst, and then supplying the stream from the catalyst recovery column including the catalyst and the extract including hydroxypivaldehyde to an aldol purification column; performing distillation to obtain a lower discharge stream including hydroxypivaldehyde and an upper discharge stream including unreacted isobutyl aldehyde and the catalyst; supplying the lower discharge stream from the aldol purification column to a hydrogenation reactor and performing a hydrogenation reaction to obtain a second reaction product including neopentyl glycol; and obtaining neopentyl glycol from the second reaction product.

Further embodiments are disclosed in the dependent claims.

### [Advantageous Effects]

According to the method for preparing neopentyl glycol of the present invention, a catalyst salt produced after an aldol condensation reaction is reduced to a catalyst by a saponification reaction and the catalyst is purified and recovered, thereby efficiently reusing the catalyst of the aldol condensation reaction. Thus, neopentyl glycol may be economically prepared and environmental pollution may be reduced.

Furthermore, in the present invention, hydroxypivalic acid-neopentyl glycol ester (HPNE) which is produced as a by-product of an aldol condensation reaction for obtaining neopentyl glycol may be recovered and obtained as a useful high value-added product, and thus, the present invention has an effect of obtaining two products by one process. Thus, production costs for preparing neopentyl glycol may be reduced to improve economic feasibility of the entire process.

### [Description of Drawings]

FIGS. 1 and 2 are process flow diagrams showing a method for preparing neopentyl glycol according to an exemplary embodiment of the present invention.
FIG. 3 is a process flow diagram showing a method for preparing neopentyl glycol according to Comparative Example 1.
FIG. 4 is a process flow diagram showing a method for preparing neopentyl glycol according to Comparative Example 2.
FIG. 5 is a process flow diagram showing a method for preparing neopentyl glycol according to Comparative Example 3.

### [Best Mode]

The terms and words used in the description and claims of the present invention are not to be construed limitedly as having general or dictionary meanings but are to be construed as having meanings and concepts meeting the technical ideas of the present invention, based on a principle that the inventors are able to appropriately define the concepts of terms in order to describe their own inventions in the best mode.

In the present invention, the term "stream" may refer to a fluid flow in a process, or may refer to a fluid itself flowing in a pipe. Specifically, the "stream" may refer to both a fluid itself flowing in a pipe connecting each device and a fluid flow. In addition, the fluid may refer to a gas or liquid, and a case in which a solid substance is included in the fluid is not excluded.

Meanwhile, in the devices such as an extraction column, a purification column, a distillation column, and a recovery column in the present invention, unless otherwise particularly stated, a "lower portion" of the device refers to a point at a height of 95% to 100% from the top to the bottom of the device, specifically the lowest part (bottom). Likewise, unless otherwise particularly stated, an "upper portion" of the device refers to, a point at a height of 0% to 5% from the top to the bottom of the device, specifically the highest part (top).

Meanwhile, in the devices such as an extraction column, a purification column, a distillation column, and a recovery column in the present invention, unless otherwise particularly stated, an operating temperature of the device may refer to a lower temperature of the device. Likewise, unless otherwise particularly stated, an operating pressure of the device may refer to upper pressure of the device.

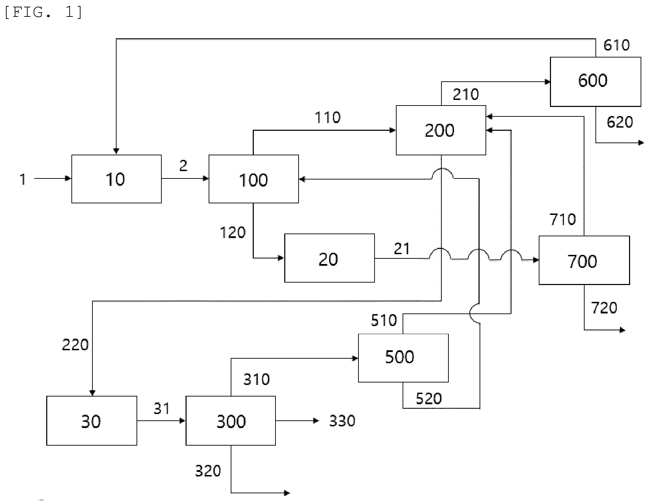

Hereinafter, the present invention will be described in more detail with reference to the FIG. 1 for better understanding of the present invention.

According to an exemplary embodiment of the present invention, a method for preparing neopentyl glycol including: performing an aldol condensation reaction of a formaldehyde aqueous solution and isobutyl aldehyde in the presence of a catalyst in an aldol reactor 10 to obtain a first reaction product including hydroxypivaldehyde; supplying the first reaction product to an aldol extraction column 100 and bringing the first reaction product into contact with an extractant to obtain an extract including hydroxypivaldehyde and a raffinate including a catalyst salt; supplying the raffinate to a saponification reactor 20 to reduce the catalyst salt to a catalyst; supplying the discharge stream 21 from the saponification reactor to a catalyst recovery column 700 to separate a stream including the catalyst, and then supplying the stream from the catalyst recovery column 700 including the catalyst and the extract including hydroxypivaldehyde to an aldol purification column 200; performing distillation to obtain a lower discharge stream 220 including hydroxypivaldehyde and an upper discharge stream 210 including unreacted isobutyl aldehyde and the catalyst; supplying the lower discharge stream 220 from the aldol purification column to a hydrogenation reactor and performing a hydrogenation reaction to obtain a second reaction product including neopentyl glycol (NPG); and obtaining neopentyl glycol from the second reaction product, is provided.

First, the method for preparing neopentyl glycol according to the present invention includes performing an aldol condensation reaction of a formaldehyde aqueous solution and isobutyl aldehyde in the presence of a catalyst in an aldol reactor 10 to obtain a first reaction product including hydroxypivaldehyde.

The aldol condensation reaction may be performed by reacting a formaldehyde (FA) aqueous solution and isobutyl aldehyde (IBAL) in the presence of the catalyst in the aldol reactor 10. Specifically, a mixed solution including a formaldehyde aqueous solution and IBAL is supplied to an aldol reactor 10 as a feed stream 1, and the aldol condensation reaction is performed in the presence of the catalyst in the aldol reactor 10 to obtain a first reaction product including hydroxypivaldehyde (HPA).

Herein, formalin may be used as the formaldehyde aqueous solution, in which a concentration of the formalin of 35 to 45 wt% may be effective in terms of waste water reduction. The formaldehyde aqueous solution may include water in an amount of 40 to 64 wt%, more specifically 45 to 55 wt% with respect to the total weight of the formaldehyde aqueous solution, and may include methanol for preventing polymerization of formaldehyde. In this case, the amount of methanol may be 0.1 to 15 wt%, more specifically 0.1 to 5 wt% with respect to the total weight of formaldehyde aqueous solution. When the amount of methanol is less than 0.1 wt%, the amount of methanol in the formaldehyde aqueous solution is insufficient, so that a polycondensation reaction in which condensation reactions are repeated may be caused. When the amount of methanol is more than 15 wt%, the amount of methanol in the formaldehyde aqueous solution is raised and the concentration of formaldehyde may be excessively lowered.

Herein, the catalyst may be an amine-based compound. Specifically, a tertiary amine compound such as trialkylamine, trimethylamine, triethylamine, tripropylamine, triisopropylamine, and tributylamine may be appropriate. More specifically, the catalyst may include triethyl amine (TEA). In the present invention, since TEA is most efficient in the aldol condensation reaction, it may be used as the catalyst.

In the aldol reactor 10, an aldol condensation reaction temperature may be 70°C to 100°C. When the aldol condensation reaction temperature is lower than 70°C, the aldol condensation reaction may not be performed well due to the low temperature, and thus, it may be difficult to obtain the first reaction product including HPA with a high conversion rate. When the aldol condensation reaction temperature is higher than 100°C, production of by-products during the aldol condensation reaction may be accelerated.

A residence time in the aldol reactor 10 may be 0.1 hours to 3 hours. When the residence time is less than 0.1 hours, a HPA yield may be decreased according to the progress of the aldol condensation reaction, and when the residence time is more than 3 hours, energy efficiency is decreased and by-products may be excessively produced as the reaction progresses for a long time.

The aldol condensation reaction proceeds under the conditions described above in the aldol reactor 10 of the present invention, thereby producing HPA. Herein, formic acid is produced by a Cannizzaro side reaction occurring in the aldol condensation reaction, and the formic acid is reacted with TEA as a catalyst to produce a TEA salt, that is, a catalyst salt. In addition, hydroxypivalic acid-neopentyl glycol ester (HPNE) may be produced by a Tishchenko reaction which is another side reaction of the aldol condensation reaction. Previously, it was common to treat the HPNE as a by-product and discard it, but according to an exemplary embodiment of the present invention, HPNE may be separated and used as a valuable raw material.

As a result, a stream 2 discharged from the aldol reactor 10 may include HPA, a catalyst salt, and HPNE as the first reaction product.

Subsequently, the method for preparing neopentyl glycol according to an exemplary embodiment of the present invention includes supplying the first reaction product to an aldol extraction column 100 and bringing the first reaction product into contact with an extractant to obtain an extract including HPA and a raffinate including a catalyst salt.

Specifically, the first reaction product including HPA produced in the aldol reactor 10 may be supplied to the aldol extraction column 100 as an aldol reactor discharge stream 2. In the aldol extraction column 100, the first reaction product supplied through the aldol reactor discharge stream 2 may be brought into contact with an extractant to obtain an organic phase extract including HPA and an extractant and a liquid raffinate including a catalyst salt. Herein, the catalyst salt may be present in a state of being dissociated in water, and the water may be derived from a formic acid aqueous solution.

Herein, the extractant may be aliphatic alcohols, and preferably, may include 2-ethylhexanol (2-EH). Since HPA included in the first reaction product is soluble in the 2-EH, it may be preferably used in the aldol extraction column 100 of the present invention which is an extraction device according to a liquid-liquid contact method described later.

An extraction device according to a liquid-liquid contact method may be used as the aldol extraction column 100. For example, the extraction device may be an extraction device such as a Karr type reciprocating plate column, a rotary-disk contactor, a Scheibel column, a spray extraction column, a packed extraction column, and a pulsed packed column.

In addition, the aldol extraction column 100 may reduce energy used in distillation in an aldol purification column 200 described later, by separating a large amount of water included in the aldol reactor discharge stream 2 as a raffinate. Herein, the water may be water included in the formaldehyde aqueous solution.

An operating temperature of the aldol extraction column 100 may be 40°C to 90°C. When the operating temperature is lower than 40°C, the first reaction product is not distilled and HPA included in the first reaction product may be cured. When the operating temperature is higher than 90°C, it may be difficult to perform phase separation of the first reaction product introduced to the aldol extraction column 100 into an organic phase and a liquid phase.

Meanwhile, according to an exemplary embodiment of the present invention, the extract may be supplied to the aldol purification column 200 as an extract stream 110 of the aldol extraction column. The extract may include unreacted IBAL and a catalyst in addition to HPA and an extractant. Herein, the unreacted IBAL may be unreacted IBAL in the aldol condensation reaction performed in the aldol reactor 10. Meanwhile, the raffinate may be supplied to a saponification reactor 20 as a raffinate stream 120 of the aldol extraction column.

Specifically, the catalyst salt included in the raffinate is reduced to a catalyst by a saponification reaction in the saponification reactor 20. The catalyst salt may be formed by a side reaction by the aldol condensation reaction as described above.

The saponification reaction may be performed by a reaction of an inorganic strong base such as sodium hydroxide (NaOH) separately added and a catalyst salt, thereby efficiently reusing a reduced catalyst. For example, when TEA is used as a catalyst of the aldol condensation reaction, a salt of TEA may be reduced to TEA by the following Reaction Formula 1:

[Reaction Formula 1] TEA-Salt + NaOH → TEA + Na-Salt

Meanwhile, in the saponification reactor 20, a temperature of the saponification reaction may be 50°C or higher, 55°C or higher, or 60°C or higher and 90°C or lower, 95°C or lower, or 100°C or lower. When the saponification reaction temperature is lower than 50°C, the saponification reaction may not be performed well due to the low temperature, and a conversion rate to the catalyst may be lowered. When the saponification reaction temperature is higher than 100°C, by-products may be excessively produced during the saponification reaction.

Meanwhile, when the catalyst salt is not reduced to the catalyst through the saponification reaction and supplied to a subsequent process, for example, a hydrogenation reactor 30 described later, in a state of a catalyst salt, a hydrogenation reaction performed in the hydrogenation reactor 30 may be adversely affected. Therefore, the catalyst salt needs to be previously reduced to the catalyst and removed and the reduced catalyst needs to be purified and recovered. Specifically, under the conditions for the hydrogenation reaction in the hydrogenation reactor 30, the catalyst salt prevents the role of a hydrogenation reaction catalyst required for a hydrogenation reaction and causes various side reactions, and thus, the conversion rate of the hydrogenation reaction may be lowered.

Therefore, the catalyst salt is reduced to the catalyst in the saponification reactor 20 and the catalyst is purified in an aldol purification column 200 described later and then recovered, thereby preventing introduction of the catalyst salt to the hydrogenation reactor 30 to minimize various side effects described above.

In addition, the catalyst salt is reduced to the catalyst in the saponification reactor 20, thereby efficiently reusing the catalyst to secure cost competitiveness of the process. Furthermore, an environmental pollution problem which may occur when the catalyst salt is not reduced and is discarded may be solved.

The catalyst reduced as such, for example, a saponification reactor discharge stream 21 including TEA may be then supplied to the aldol purification column 200.

The method for preparing neopentyl glycol according to the present invention further includes supplying the saponification reactor discharge stream 21 to a catalyst recovery column 700 to separate a stream including the catalyst, and then supplying the stream from the catalyst recovery unit 700 including the catalyst and the extract including hydroxypivaldehyde to an aldol purification column 200.

More specifically, the saponification reactor discharge stream 21 is supplied to the catalyst recovery column 700 before it is supplied to the aldol purification column 200. In the catalyst recovery column 700, the saponification reactor discharge stream 21 including the catalyst may be distilled to be separated into an upper fraction including a reduced catalyst and a lower fraction including water and by-products.

The upper fraction of the catalyst recovery column 700 may be supplied to the aldol purification column 200 as an upper discharge stream 710 from the catalyst recovery column. Meanwhile, the lower fraction of the catalyst recovery column 700 may be discharged as a lower discharge stream 720 from the catalyst recovery column. As such, water and by-products are separated in the catalyst recovery column 700, thereby recovering the reduced catalyst. As such, in the catalyst recovery column 700, a large amount of water and by-products are removed, and the reduced catalyst is recovered and supplied to the aldol purification column 200, thereby significantly reducing an amount of energy used for distilling water in the aldol purification column 200.

The method for preparing neopentyl glycol according to the present invention includes performing distillation to obtain a lower discharge stream 220 from the aldol purification column including hydroxypivaldehyde and an upper discharge stream 210 from the aldol purification column including unreacted isobutyl aldehyde and the catalyst.

Herein, the discharge stream 21 from the saponification reactor including the catalyst is a stream discharged from the saponification reactor, and the catalyst may be a catalyst reduced from the catalyst salt by a saponification reaction performed in the saponification reactor. The discharge stream 21 from the saponification reactor passes through a catalyst recovery column 700 to remove water and by-products therefrom as described above and is supplied to the aldol purification column 200.

Specifically, in the aldol purification column 200, a supply supplied to the aldol purification column 200 may be distilled to obtain the lower discharge stream 220 from the aldol purification column including HPA and the extractant and the upper discharge stream 210 from the aldol purification column including unreacted IBAL and the catalyst. The supply supplied to the aldol purification column 200 may further include an upper discharge stream 510 from an extractant recovery column described later, in addition to the extract and an upper discharge stream 710 from the catalyst recovery column.

Meanwhile, an operating temperature of the aldol purification column 200 may be 40°C or higher, 45°C or higher, 50°C or higher, or 55°C or higher and 85°C or lower, 90°C or lower, 95°C or lower, or 100°C or lower.

In addition, an operating pressure of the aldol purification column 200 may be or more, 46.66 kPa (350 torr) or more, 53.33 kPa (400 torr) or more, or kPa (450 torr) or more and 80 kPa (600 torr) or less, 86.66 kPa (650 torr) or less, 93.33 kPa (700 torr) or less, or 101.33 kPa (760 torr) or less. By operating the aldol purification column 200 within the ranges of temperature and pressure, distillation is performed well, so that separation of TEA which is a relatively low-boiling point material and HPA which is a relatively high-boiling point material may be easily performed.

According to the present invention, by subjecting the catalyst to a purification process in the aldol purification column 200, a higher-purity catalyst may be recovered, which may be reused in an aldol condensation reaction. Thus, efficiency of the aldol condensation reaction may be improved as compared with the conventional case of supplying a reduced catalyst to the aldol reactor 10 without a separate purification process, and production of by-products by a side reaction may be minimized. In addition, a separate purification process of the reduced catalyst is performed, thereby decreasing an amount of a liquid phase supplied to the aldol purification column 200, for example, water, to decrease an amount of energy used in the aldol purification column 200.

In addition, unreacted IBAL in the aldol condensation reaction is separated as an extract of an aldol extraction column 100, the separated extract may be supplied to the aldol purification column 200 as described above, and unreacted IBAL purified in the aldol purification column 200 may be separated as an upper fraction of the aldol purification column 200 with the catalyst. The upper fraction of the aldol purification column 200 may be circulated to the aldol reactor 10 through the upper discharge stream 210 from the aldol purification column.

According to an exemplary embodiment of the present invention, the upper fraction of the aldol purification column 200 including the unreacted IBAL and the catalyst may be supplied to a raw material recovery column 600 through the upper discharge stream 210 from the aldol purification column. In the raw material recovery column 600, the upper discharge stream 210 from the aldol purification column including the unreacted IBAL and the catalyst may be distilled to be separated into an upper fraction including the unreacted IBAL and the catalyst and a lower fraction including water and by-products as impurities.

The upper fraction of the raw material recovery column 600 may be circulated to the aldol reactor 10 as an upper discharge stream 610 from the raw material recovery column. Meanwhile, the lower fraction of the raw material recovery column 600 may be discharged as a lower discharge stream 620 from the raw material recovery column. By separating the unreacted IBAL and the catalyst once again from impurities in the raw material recovery column 600 as such, it may be appropriate for reuse as a raw material of the aldol condensation reaction in the aldol reactor 10. In addition, since the catalyst and IBAL used in the aldol reactor 10 is reused, the amount of a newly added raw material may be reduced and production costs used during the process may be reduced.

The method for preparing neopentyl glycol according to the present invention includes supplying the lower discharge stream 220 from the aldol purification column to a hydrogenation reactor 30 and performing a hydrogenation reaction to obtain a second reaction product including NPG. Herein, the lower discharge stream 220 from the aldol purification column may include HPA and the extractant.

Meanwhile, a hydrogenation reaction may proceed in the hydrogenation reactor 30, and the hydrogenation reaction may be performed by a reaction of hydrogen which is further added to the hydrogenation reactor 30 and HPA in the presence of a hydrogenation reaction catalyst.

The hydrogenation reaction may proceed at a hydrogen pressure of 689.476 to 10342.14 kPa (100 to 1500 psig, pounds per square inch gauge pressure) and a reaction temperature of 100°C to 200°C. In addition, a copper-based catalyst or a nickel catalyst may be used as the hydrogenation reaction catalyst. The copper-based catalyst may be, for example, a CuO/BaO/SiO catalyst, and the CuO/BaO/SiO catalyst may be a catalyst of (CuO)ₓ(BaO)_{y}(SiO)_{z} (x, y, and z are wt%, and x:y:z = 10-50:0-10:40-90, 10-50:1-10:40-89, or 29-50:1-10:40-70). The sum of x and y is preferably 20 to 50 (wt%) or 30 to 50 (wt%), based on the sum of x, y, and z (100 wt%), and in this range, performance of the hydrogenation reaction catalyst is excellent and an effect of long life is shown. Meanwhile, the nickel catalyst may be 2 to 10 wt% with respect to the weight of HPA.

As described above, when a catalyst salt formed by the aldol condensation reaction, for example, a TEA salt, is introduced to the hydrogenation reaction, various side reactions may be caused to decrease a conversion rate of the hydrogenation reaction. Therefore, TEA converted by the saponification reactor 20 of the present invention is purified and recovered in the aldol purification column 200, thereby improving the conversion rate of the hydrogenation reaction in the hydrogenation reactor 30. That is, an inflow amount of a TEA salt causing a poisoning phenomenon for a hydrogenation reaction catalyst, that is, a catalyst poison, is decreased, thereby activating the hydrogenation reaction.

As such, the hydrogenation reaction proceeds in the hydrogenation reactor 30, and when HPA reacts with hydrogen, NPG may be produced. Therefore, a second reaction product including the catalyst, the extractant, HPNE, and the NPG may be obtained. Herein, the HPNE may be produced as a by-product of an aldol condensation reaction in the aldol reactor 10.

The method for preparing neopentyl glycol according to the present invention includes obtaining NPG from the second reaction product.

Specifically, the second reaction product may include the catalyst, neopentyl glycol, the extractant, and hydroxypivalic acid-neopentyl glycol ester (HPNE), and the obtaining the neopentyl glycol from the second reaction product may be performed by including supplying the second reaction product to a NPG purification column 300, supplying a stream including the catalyst and the extractant to an extractant recovery column 500 and supplying a stream including HPNE to a HPNE purification column 400, and obtaining NPG from a stream including NPG; and distilling a stream including the HPNE in the HPNE purification column 400 to obtain HPNE.

Specifically, the second reaction product may be supplied to a neopentyl glycol (NPG) purification column 300 as a discharge stream 31 from the hydrogenation reactor. Herein, the NPG purification column 300 may be one or two or more purification columns.

First, when the NPG purification column 300 is one purification column, the extractant and the catalyst may be separated from an upper portion, HPNE may be separated from a lower portion, and NPG may be separated from a side portion of the one purification column, respectively. For example, the NPG purification column 300 may be one separation wall-type distillation column. Meanwhile, when the NPG purification column 300 is two or more purification columns, it may be performed through one or more NPG purification columns which separate the catalyst into the upper portion and one or more NPG purification columns which separate the extractant into the upper portion. A stream including HPNE or NPG may be separated and discharged into the lower portion of two or more NPG purification columns. That is, the second reaction product may be separated into the catalyst and the extractant, HPNE, and NPG by the one or two or more NPG purification columns 300. Thus, high-purity NPG may be obtained.

Meanwhile, when the NPG purification column 300 is one purification column, neopentyl glycol may be obtained from the side portion at a height of 40% to 80% from the top to the bottom of the neopentyl glycol purification column 300.

Meanwhile, an operating temperature of the NPG purification column 300 may be 80°C or higher, 100°C or higher, 120 °C or higher, or 140°C or higher and 185°C or lower, 190°C or lower, 195°C or lower, or 200°C or lower. In addition, an operating pressure of the NPG purification column 300 may be 5.33 kPa (40 torr) or more, 12 kPa (90 torr) or more, 16 kPa (120 torr) or more, or 18.67 kPa (140 torr) more and 40 kPa (300 torr) or less, 53.33 kPa (400 torr) or less, 66.66 kPa (500 torr) or less, or 80 kPa (600 torr) or less. By operating the NPG purification column 300 within the temperature and pressure ranges, it may be easy to separate the extractant and the catalyst, NPG, and HPNE, as described above. Therefore, since the amount of by-products present in the stream 330 including NPG to be obtained in the present invention may be decreased, high-purity NPG may be obtained.

Meanwhile, as the HPNE produced by the side reaction of the aldol condensation reaction is partly reduced to NPG in the hydrogenation reactor 30 and the rest remains as HPNE, the HPNE is introduced to the NPG purification column 300 to decrease a NPG yield. Therefore, the HPNE is supplied to a HPNE purification column 400 described later, thereby further obtaining NPG and also separately recovering the HPNE which is a high value-added product itself and using it. That is, the HPNE may be distinguished from other heavy by-products, for example, trimethylpentanediol (2,2,4-trimethyl-1,3-pentanediol; TMPD) which is a by-product of the hydrogenation reaction and commercialized.

Meanwhile, according to an exemplary embodiment of the present invention, a stream 310 including the catalyst and the extractant may be supplied to an extractant recovery column 500. In the extractant recovery column 500, an upper fraction including the catalyst and a lower fraction including the extractant may be separated.

Herein, the catalyst may be some small amounts of catalyst which is not separated from the aldol extraction column 100 and the aldol purification column 200 described above, and the catalyst is separated from the extractant recovery column 500 and supplied to the aldol purification column 200, thereby recovering the catalyst in the system, for example, TEA.

Meanwhile, the lower fraction of the extractant recovery column 500 including the extractant is supplied to the aldol extraction column 100 as a lower discharge stream 520 from the extractant recovery column, and the extractant, for example, 2-EH may be separated and recovered therefrom and reused.

An operating temperature of the extractant recovery column 500 may be 30°C or higher, 35°C or higher, or 40°C or higher and 165°C or lower, 170°C or lower, or 180°C or lower. In addition, an operation pressure of the extractant recovery column 500 may be 13.33 kPa (100 torr) or more, 14.67 kPa (110 torr) or more, or 17.33 kPa (130 torr) or more and 50.66 kPa (380 torr) or less, 53.33 kPa (400 torr) or less, or 60 kPa (450 torr) or less. By operating the extractant recovery column 500 within the temperature and pressure ranges, separation of the catalyst and the extractant may be performed well.

Meanwhile, in the present invention, a stream 320 including the HPNE may be supplied to a HPNE purification column 400. The stream 320 including the HPNE may be distilled in the HPNE purification column 400 to be separated into NPG, HPNE, and high-boiling point by-products (heavies). The separated high-boiling point by-products may be discharged as a HPNE lower discharge stream 420.

In addition, the HPNE separated from a side discharge stream 430 from the HPNE purification column may be obtained. The obtained HPNE may be recovered and used in various ways as described above, and for example, may be used as a main raw material of synthesizing and coating polyester as a high value-added product. As such, since the HPNE may be used as a raw material in other processes, economic feasibility in terms of raw material use may be improved by the HPNE purification column 400 according to the present invention.

The HPNE may be obtained from the side portion at a height of 50% to 80% from the top to the bottom of the HPNE purification column. Specifically, by obtaining HPNE from the side portion at a height within the range, an amount of HPNE in the side discharge stream 430 from the HPNE purification column may be high, and thus, it may be preferred for obtaining HPNE therefrom. Meanwhile, when HPNE is obtained from the side portion out of the height within the range, an amount of NPG in the side discharge stream 430 from the HPNE purification column may be increased or an amount of high-boiling point by-products may be increased, as compared with the case in which the side portion belongs to the height within the range.

An operating temperature of the HPNE purification column may be 100°C or higher, 110°C or higher, or 120°C or higher and 230°C or lower, 240°C or lower, or 250°C or lower. In addition, an operation pressure of the HPNE purification column may be 5.33 kPa(40 torr) more, 6 kPa (45 torr) or more, or 6.67 kPa (50 torr) or more and 20 kPa(150 torr) or less, 21.33 kPa (160 torr) or less, or 22.66 kPa (170 torr) or less. By operating the HPNE purification column 400 within the temperature and pressure ranges, separation of HPNE and NPG may be performed well to further obtain NPG to be obtained in the present invention, and HPNE may be also obtained as a high value-added product.

The method for preparing neopentyl glycol of the present invention may further include recovering NPG from an upper discharge stream 410 from the HPNE purification column and refluxing NPG to a NPG purification column 300. That is, since NPG which is not recovered in the NPG purification column 300 and discharged may be recovered from the upper portion 410 of the HPNE purification column 400, a NPG recovery rate may be further increased as compared with a conventional method for preparing NPG which is not provided with the HPNE purification column 400.

Hereinafter, the present invention will be described in more detail by the examples.

### Examples

### Example 1

According to the process flow illustrated in FIG. 1, a preparation process of neopentyl glycol (NPG) was simulated, using an Aspen Plus simulator available from Aspen.

Specifically, an aldol condensation reaction of a formaldehyde aqueous solution and isobutyl aldehyde was performed in the presence of a catalyst (triethylamine, TEA) in an aldol reactor 10 to obtain a first reaction product including hydroxypivaldehyde (HPA). At this time, the aldol condensation reaction was performed at a temperature of 85°C.

The first reaction product was supplied to the aldol extraction column 100 and brought into contact with an extractant (2-ethylhexanol; 2-EH) to obtain an extract including hydroxypivaldehyde and a raffinate including a catalyst salt.

The raffinate was supplied to a saponification reactor 20 as a raffinate stream 120, the catalyst salt was reacted with sodium hydroxide (NaOH) to be reduced to the catalyst, and a discharge stream 21 from the saponification reactor including the reduced catalyst was supplied to a catalyst recovery column 700. At this time, a saponification reaction performed in the saponification reactor was performed at a temperature of 83°C.

The discharge stream 21 from the saponification reactor was distilled in the catalyst recovery column 700 to remove water and by-products, and a stream including the catalyst from which water and by-products had been removed was obtained. The stream including the catalyst from which water and by-products had been removed was supplied to an aldol purification column 200 as an upper discharge stream 710 from a catalyst recovery column.

An extract stream 110 including the extract described above and the discharge stream 710 from the catalyst recovery column was supplied to the aldol purification column 200 and distilled to obtain a lower discharge stream 220 including HPA and an upper discharge stream 210 including unreacted isobutyl aldehyde and the catalyst. The operating pressure of the aldol purification column 200 was 27.60 kPa (207 torr) and the operating temperature thereof was 87°C. Meanwhile, the upper discharge stream 210 including the unreacted isobutyl aldehyde and the catalyst was supplied to a raw material recovery column 600.

Impurities including water and by-products were removed and a stream including the unreacted isobutyl aldehyde and the catalyst from which impurities had been removed was obtained, by distillation performed in the raw material recovery column 600. The stream including unreacted isobutyl aldehyde and the catalyst from which impurities had been removed was supplied to the aldol reactor 10 as an upper discharge stream 610 from the raw material recovery column.

Meanwhile, a lower discharge stream 220 from the aldol purification column was supplied to a hydrogenation reactor 30 and hydrogenated to obtain a second reaction product including NPG. At this time, the hydrogenation reaction was performed at a temperature of 160°C.

The second reaction product was supplied to a neopentyl glycol purification column 300 to obtain an upper discharge stream 310 from the NPG purification column including the catalyst and the extractant and a lower discharge stream 320 from the NPG purification column including HPNE, and NPG was obtained from a side discharge stream 330 from the NPG purification column including NPG. The side discharge stream 330 from the NPG purification column was discharged from a side portion at a height of 20 to 70% from the top to the bottom of the NPG purification column 300. At this time, the operating pressure of the neopentyl glycol purification column was 20.53 kPa (154 torr) and the operating temperature thereof was 168°C.

The upper discharge stream 310 from the NPG purification column was supplied to an extractant recovery column 500 and distilled, and an upper discharge stream 510 from the extractant recovery column including the catalyst was supplied to the aldol purification column 200 and a lower discharge stream 520 from the extractant recovery column including the extractant was supplied to the aldol extraction column 100.

At this time, energy used in the aldol purification column 200 was measured, and when it was converted based on energy used in the aldol purification column of Comparative Example 1, it was confirmed that an energy use rate of the aldol purification column was 86.20%.

### Example 2

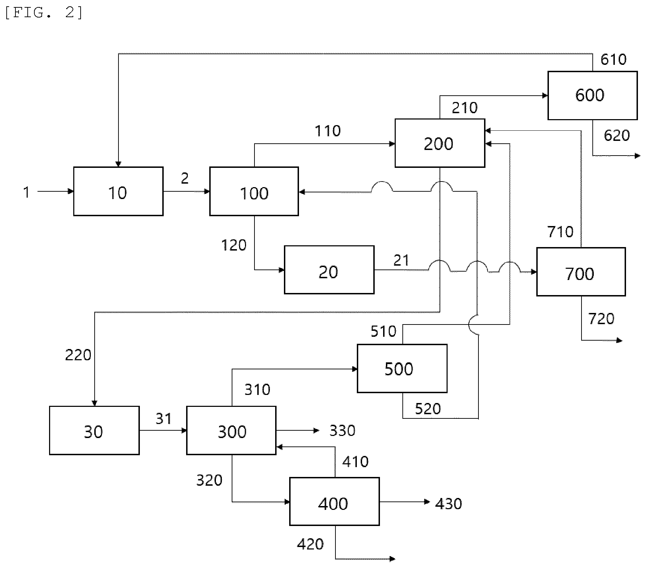

According to the process flow illustrated in FIG. 2, a preparation process of neopentyl glycol (NPG) was simulated, using the Aspen Plus simulator available from Aspen.

In Example 2, as compared with Example 1, a HPNE purification column 400 was further provided, and NPG was prepared in the same process flow as in Example 1, except that the lower discharge stream 320 from the NPG purification column was supplied to the HPNE purification column 400.

Specifically, the lower discharge stream 320 from the NPG purification column including HPNE was supplied to the HPNE purification column 400, the upper discharge stream 410 from the HPNE purification column including NPG was refluxed to the NPG purification column 300, and the lower discharge stream 420 from the HPNE purification column including high-boiling point by-products was discharged out of the system. In addition, HPNE was separately obtained from a side discharge stream 430 from the HPNE purification column including HPNE.

### Comparative Examples

### Comparative Example 1

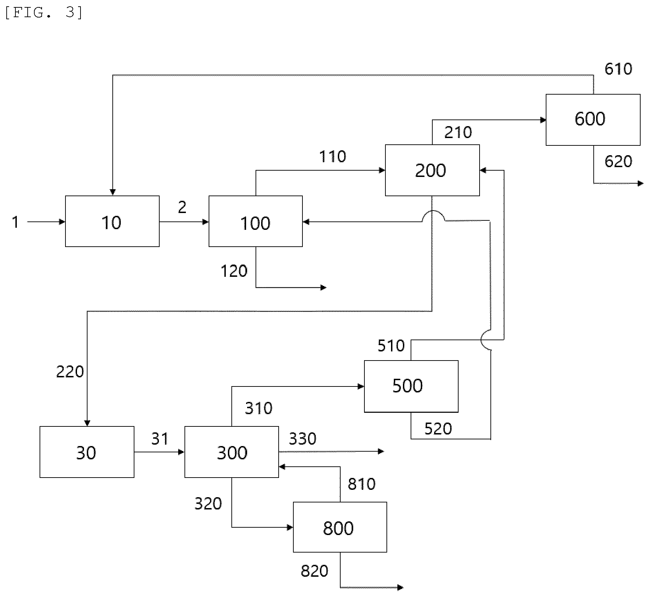

According to the process flow illustrated in FIG. 3, a preparation process of neopentyl glycol (NPG) was simulated, using the Aspen Plus simulator available from Aspen.

In Comparative Example 1, NPG was prepared in the same process flow as in Example 1, except that since the saponification reactor was not provided, the lower discharge stream 120 from the aldol extraction column was discharged out of the system, the lower discharge stream 320 from the NPG purification column was supplied to a high-boiling point by-product separation column 800, not the HPNE purification column.

Specifically, the lower discharge stream 320 from the NPG purification column was supplied to the high-boiling point by-product separation column 800 and distilled to obtain an upper discharge stream 810 including NPG and a lower discharge stream 820 including high-boiling point by-products and HPNE. At this time, the HPNE was not separately obtained, the upper discharge stream 810 from the high-boiling point by-product separation column was circulated to the NPG purification column 300.

At this time, energy used in the aldol purification column 200 was measured, which was set as 100%.

### Comparative Example 2

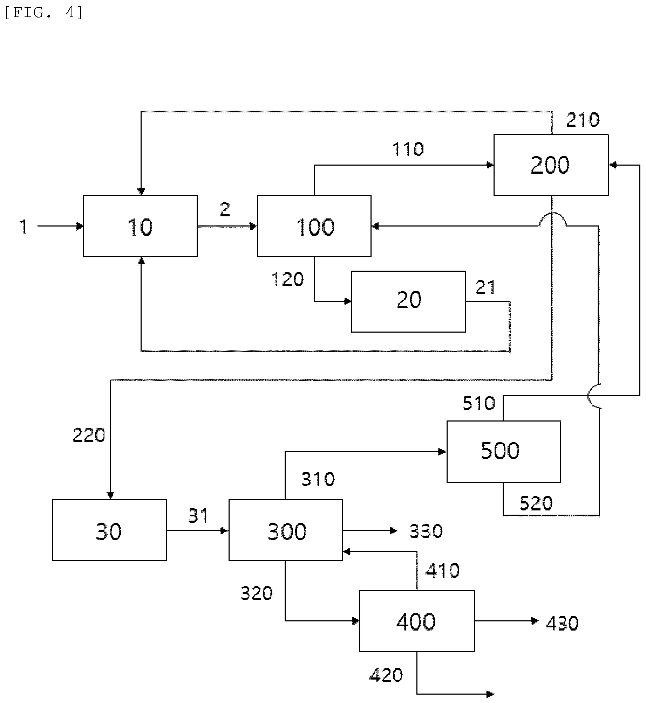

According to the process flow diagram illustrated in FIG. 4, a preparation process of neopentyl glycol (NPG) was simulated, using the Aspen Plus simulator available from Aspen.

In Comparative Example 2, NPG was prepared in the same process flow as in Example 2, except that the discharge stream 21 from the saponification reactor was supplied to the aldol reactor 10 without passing through the catalyst recovery column, and the upper discharge stream 210 from the aldol purification column was supplied to the aldol reactor 10 without passing through the raw material recovery column.

### Comparative Example 3

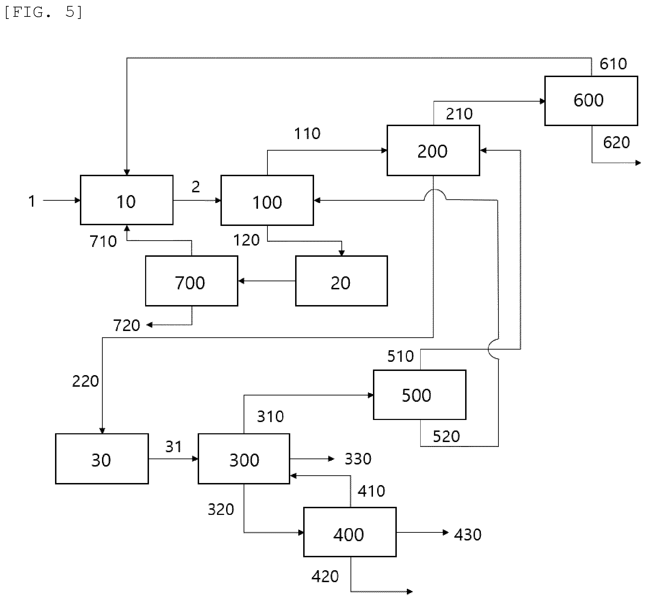

According to the process flow diagram illustrated in FIG. 5, a preparation process of neopentyl glycol (NPG) was simulated, using the Aspen Plus simulator available from Aspen.

In Comparative Example 3, NPG was prepared in the same process flow as in Example 2, except that the upper discharge stream 710 from the catalyst recovery column was not supplied to the aldol purification column, but was condensed and supplied to the aldol reactor 10.

In the following Table 1, a fresh TEA supply rate, a TEA loss rate, and an energy use rate of the aldol purification column of the examples and the comparative examples were converted based on Comparative Example 1 as 100% and shown, respectively. Specifically, the fresh TEA supply rate shows the weight of TEA newly supplied into the process of each of the examples and the comparative examples to the weight of TEA newly supplied into the process of Comparative Example 1, as a percentage. Meanwhile, the TEA loss rate shows the amount of a TEA salt which was not reduced to TEA through the saponification reactor, based on Comparative Example 1. Meanwhile, the energy use rate of the aldol purification column shows the amount of energy used in the aldol purification column of each of the examples and the comparative examples to the amount of energy used in the aldol purification column of Comparative Example 1, as a percentage.

**[Table 1]**

| | Fresh TEA supply rate (wt%) | TEA loss rate (wt%) | HPNE product | Energy use rate of aldol purification column (%) |
|---|---|---|---|---|
| Example 1 | 18.1 | 0 | × | 86.20 |
| Example 2 | 18.1 | 0 | ○ | 86.20 |
| Comparative Example 1 | 100 | 100 | × | 100.00 |
| Comparative Example 2 | 18.1 | 0 | ○ | 139.00 |
| Comparative Example 3 | 18.1 | 0 | ○ | 109.28 |

Referring to Table 1, it was confirmed that the examples had good fresh TEA supply rates and good energy use rates of the aldol purification column. Since Example 2 was not provided with the HPNE purification column, it was confirmed that the HPNE product was not able to be further obtained.

Meanwhile, since Comparative Example 1 was not provided with the saponification reactor, the catalyst salt present in the lower discharge stream from the aldol extraction column was not reduced to the catalyst and reused, and thus, it was confirmed that the supply amount of new TEA and the TEA loss amount were highest. In addition, the high-boiling point by-product separation column was provided instead of the HPNE purification column, and thus, the HPNE product was not able to be further obtained. In addition, it was confirmed that the aldol purification column energy use rate was high as compared with those of the examples.

Meanwhile, in Comparative Example 2, the discharge stream from the saponification reactor was supplied to the aldol reactor without passing through the catalyst recovery column, and it was confirmed that the aldol purification column energy use rate was the highest. Since the amount of water in the discharge stream from the saponification reactor was very high, when it was directly supplied to the aldol reactor, the amount of water introduced to the aldol purification column was also increased, and thus, the energy use amount in the aldol purification column for distilling water was able to be increased.

Meanwhile, in Comparative Example 3, the upper discharge stream from the catalyst recovery column was supplied to the aldol reactor, not the aldol purification column, and it was confirmed that the aldol purification column energy use rate was higher than those of the examples and Comparative Example 1. Specifically, since the upper discharge stream from the catalyst recovery column was a gas phase, in order to supply it to the aldol reactor, the gas phase upper discharge stream from the catalyst recovery column was condensed into a liquid phase and supplied to the aldol reactor. When the upper discharge stream from the catalyst recovery column condensed into a liquid phase was supplied to the aldol reactor as such, it was able to be introduced to the aldol purification column after the aldol condensation reaction in the aldol reactor, and thus, was able to increase the energy usage in the aldol purification column.

## Claims

1. A method for preparing neopentyl glycol, the method comprising:
performing an aldol condensation reaction of a formaldehyde aqueous solution and isobutyl aldehyde in the presence of a catalyst in an aldol reactor to obtain a first reaction product including hydroxypivaldehyde;
supplying the first reaction product to an aldol extraction column and bringing the first reaction product into contact with an extractant to obtain an extract including hydroxypivaldehyde and a raffinate including a catalyst salt;
supplying the raffinate to a saponification reactor to reduce the catalyst salt to a catalyst;
supplying the discharge stream from the saponification reactor to a catalyst recovery column to separate a stream including the catalyst, and then supplying the stream from the catalyst recovery column including the catalyst and the extract including hydroxypivaldehyde to an aldol purification column;
performing distillation to obtain a lower discharge stream from the aldol purification column including hydroxypivaldehyde and an upper discharge stream from the aldol purification column including unreacted isobutyl aldehyde and the catalyst;
supplying the lower discharge stream from the aldol purification column to a hydrogenation reactor and performing a hydrogenation reaction to obtain a second reaction product including neopentyl glycol; and
obtaining neopentyl glycol from the second reaction product.

2. The method for preparing neopentyl glycol of claim 1, further comprising: circulating the upper discharge stream from the aldol purification column to the aldol reactor.

3. The method for preparing neopentyl glycol of claim 2, further comprising:
supplying the upper discharge stream from the aldol purification column to a raw material recovery column and distilling the upper discharge stream from the aldol purification column and
circulating an upper discharge stream from the raw material recovery column including the unreacted isobutyl aldehyde and the catalyst to the aldol reactor.

4. The method for preparing neopentyl glycol of claim 1,
wherein the second reaction product includes the catalyst, neopentyl glycol, the extractant, and hydroxypivalic acid-neopentyl glycol ester (HPNE), and
the obtaining of neopentyl glycol from the second reaction product further includes:
supplying the second reaction product to the neopentyl glycol purification column, supplying a stream including the catalyst and the extractant to an extractant recovery column, supplying a stream including hydroxypivalic acid-neopentyl glycol ester to a hydroxypivalic acid-neopentyl glycol ester purification column, and obtaining neopentyl glycol from a stream including neopentyl glycol; and
distilling the stream including the hydroxypivalic acid-neopentyl glycol ester in the hydroxypivalic acid-neopentyl glycol ester purification column to obtain hydroxypivalic acid-neopentyl glycol ester.

5. The method for preparing neopentyl glycol of claim 4, which includes recovering the catalyst from an upper fraction of the extractant recovery column and supplying the catalyst to the aldol purification column, and
recovering the extractant from a lower fraction of the extractant recovery column and supplying the extractant to the aldol extraction column.

6. The method for preparing neopentyl glycol of claim 4, which includes recovering neopentyl glycol from an upper discharge stream from the hydroxypivalic acid-neopentyl glycol ester purification column and refluxing neopentyl glycol to a neopentyl glycol purification column.

7. The method for preparing neopentyl glycol of claim 1, wherein the catalyst includes triethyl amine (TEA).

8. The method for preparing neopentyl glycol of claim 1, wherein the extractant includes 2-ethylhexanol (2-EH).

9. The method for preparing neopentyl glycol of claim 4, wherein the hydroxypivalic acid-neopentyl glycol ester is obtained from a side portion at a height of 50 to 80% from the top to the bottom of the hydroxypivalic acid-neopentyl glycol ester purification column.

## Patentansprüche

1. Verfahren zur Herstellung von Neopentylglykol, wobei das Verfahren umfasst:
Durchführen einer Aldolkondensationsreaktion einer wässrigen Formaldehydlösung und von Isobutylaldehyd in Gegenwart eines Katalysators in einem Aldolreaktor, um ein erstes Reaktionsprodukt zu erhalten, das Hydroxypivaldehyd enthält;
Zuführen des ersten Reaktionsprodukts zu einer Aldolextraktionssäule und Inkontaktbringen des ersten Reaktionsprodukts mit einem Extraktionsmittel, um einen Extrakt, der Hydroxypivaldehyd enthält, und ein Raffinat, das ein Katalysatorsalz enthält, zu erhalten;
Zuführen des Raffinats zu einem Verseifungsreaktor, um das Katalysatorsalz zu einem Katalysator zu reduzieren;
Zuführen des Austragsstroms aus dem Verseifungsreaktor zu einer Katalysatorrückgewinnungssäule, um einen Strom, der den Katalysator enthält, abzutrennen, und dann Zuführen des Stroms aus der Katalysatorrückgewinnungssäule, der den Katalysator und den Extrakt, der Hydroxypivaldehyd enthält, enthält, zu einer Aldolreinigungssäule;
Durchführen einer Destillation, um einen unteren Austragsstrom aus der Aldolreinigungssäule, der Hydroxypivaldehyd enthält, und einen oberen Austragsstrom aus der Aldolreinigungssäule, der nicht umgesetzten Isobutylaldehyd und den Katalysator enthält, zu erhalten;
Zuführen des unteren Austragsstroms aus der Aldolreinigungssäule zu einem Hydrierungsreaktor und Durchführen einer Hydrierungsreaktion, um ein zweites Reaktionsprodukt, das Neopentylglykol enthält, zu erhalten; und
Erhalten von Neopentylglykol aus dem zweiten Reaktionsprodukt.

2. Verfahren zur Herstellung von Neopentylglykol nach Anspruch 1, ferner umfassend: Zirkulieren des oberen Austragsstroms aus der Aldolreinigungssäule zu dem Aldolreaktor.

3. Verfahren zur Herstellung von Neopentylglykol nach Anspruch 2, ferner umfassend:
Zuführen des oberen Austragsstroms aus der Aldolreinigungssäule zu einer Rohmaterialrückgewinnungssäule und Destillieren des oberen Austragsstroms aus der Aldolreinigungssäule und
Zirkulieren eines oberen Austragsstroms aus der Rohmaterialrückgewinnungssäule, der den nicht umgesetzten Isobutylaldehyd und den Katalysator enthält, zu dem Aldolreaktor.

4. Verfahren zur Herstellung von Neopentylglykol nach Anspruch 1,
wobei das zweite Reaktionsprodukt den Katalysator, Neopentylglykol, das Extraktionsmittel und Hydroxypivalinsäure-Neopentylglykolester (HPNE) enthält, und
das Erhalten von Neopentylglykol aus dem zweiten Reaktionsprodukt ferner umfasst:
Zuführen des zweiten Reaktionsprodukts zu der Neopentylglykolreinigungssäule, Zuführen eines Stroms, der den Katalysator und das Extraktionsmittel enthält, zu einer Extraktionsmittelrückgewinnungssäule, Zuführen eines Stroms, der Hydroxypivalinsäure-Neopentylglykolester enthält, zu einer Hydroxypivalinsäure-Neopentylglykolesterreinigungssäule und Erhalten von Neopentylglykol aus einem Strom, der Neopentylglykol enthält; und
Destillieren des Stroms, der den Hydroxypivalinsäure-Neopentylglykolester enthält, in der Hydroxypivalinsäure-Neopentylglykolesterreinigungssäule, um Hydroxypivalinsäure-Neopentylglykolester zu erhalten.

5. Verfahren zur Herstellung von Neopentylglykol nach Anspruch 4, das das Rückgewinnen des Katalysators aus einer oberen Fraktion der Extraktionsmittelrückgewinnungssäule und das Zuführen des Katalysators zu der Aldolreinigungssäule und
das Rückgewinnen des Extraktionsmittels aus einer unteren Fraktion der Extraktionsmittelrückgewinnungssäule und das Zuführen des Extraktionsmittels zu der Aldolextraktionssäule umfasst.

6. Verfahren zur Herstellung von Neopentylglykol nach Anspruch 4, das das Rückgewinnen von Neopentylglykol aus einem oberen Austragsstrom aus der Hydroxypivalinsäure-Neopentylglykolesterreinigungssäule und das Refluxieren von Neopentylglykol zu einer Neopentylglykolreinigungssäule umfasst.

7. Verfahren zur Herstellung von Neopentylglykol nach Anspruch 1, wobei der Katalysator Triethylamin (TEA) enthält.

8. Verfahren zur Herstellung von Neopentylglykol nach Anspruch 1, wobei das Extraktionsmittel 2-Ethylhexanol (2-EH) enthält.

9. Verfahren zur Herstellung von Neopentylglykol nach Anspruch 4, wobei der Hydroxypivalinsäure-Neopentylglykolester von einem Seitenabschnitt in einer Höhe von 50 bis 80 % von der Oberseite zu der Unterseite der Hydroxypivalinsäure-Neopentylglykolesterreinigungssäule erhalten wird.

## Revendications

1. Procédé de préparation de néopentylglycol, le procédé consistant à :
effectuer une réaction de condensation aldolique d'une solution aqueuse de formaldéhyde et d'aldéhyde isobutyle en présence d'un catalyseur dans un réacteur d'aldol pour obtenir un premier produit de réaction incluant de l'hydroxypivaldéhyde ;
fournir le premier produit de réaction à une colonne d'extraction d'aldol et amener le premier produit de réaction en contact avec un extractant pour obtenir un extrait incluant de l'hydroxypivaldéhyde et un raffinat incluant un sel de catalyseur ;
fournir le raffinat à un réacteur de saponification pour réduire le sel de catalyseur à un catalyseur ;
fournir le flux d'évacuation du réacteur de saponification à une colonne de récupération de catalyseur pour séparer un flux incluant le catalyseur, puis fournir le flux de la colonne de récupération de catalyseur incluant le catalyseur et l'extrait incluant de l'hydroxypivaldéhyde à une colonne de purification d'aldol ;
effectuer une distillation pour obtenir un flux d'évacuation inférieur provenant de la colonne de purification d'aldol incluant de l'hydroxypivaldéhyde et un flux d'évacuation supérieur de la colonne de purification d'aldol incluant de l'aldéhyde isobutyle non réagi et le catalyseur ;
fournir le flux d'évacuation inférieur provenant de la colonne de purification d'aldol à un réacteur d'hydrogénation et effectuer une réaction d'hydrogénation pour obtenir un deuxième produit de réaction incluant du néopentylglycol ; et
obtenir du néopentylglycol du deuxième produit de réaction.

2. Procédé de préparation de néopentylglycol selon la revendication 1, consistant en outre à : faire circuler le flux d'évacuation supérieur de la colonne de purification d'aldol vers le réacteur d'aldol.

3. Procédé de préparation de néopentylglycol selon la revendication 2, consistant en outre à :
fournir le flux d'évacuation supérieur de la colonne de purification d'aldol à une colonne de récupération de matière brute et distiller le flux d'évacuation supérieur de la colonne de purification d'aldol et
faire circuler un flux d'évacuation supérieur de la colonne de récupération de matière brute incluant l'aldéhyde isobutyle non réagi et le catalyseur vers le réacteur d'aldol.

4. Procédé de préparation de néopentylglycol selon la revendication 1,
dans lequel le deuxième produit de réaction inclut le catalyseur, du néopentylglycol, l'extractant et l'acide hydroxypivalique-ester de néopentylglycol (HPNE), et
l'obtention du néopentylglycol du deuxième produit de réaction consiste en outre à :
fournir le deuxième produit de réaction à la colonne de purification de néopentylglycol, fournir un flux incluant le catalyseur et l'extractant à une colonne de récupération d'extractant, fournir un flux incluant l'acide hydroxypivalique-ester de néopentylglycol à une colonne de purification d'acide hydroxypivalique-ester de néopentylglycol, et obtenir le néopentylglycol d'un flux incluant le néopentylglycol ; et
distiller le flux incluant l'acide hydroxypivalique-ester de néopentylglycol dans la colonne de purification d'acide hydroxypivalique-ester de néopentylglycol pour obtenir l'acide hydroxypivalique-ester de néopentylglycol.

5. Procédé de préparation de néopentylglycol selon la revendication 4, lequel consiste à récupérer le catalyseur d'une fraction supérieure de la colonne de récupération d'extractant et à fournir le catalyseur à la colonne de purification d'aldol, et
récupérer l'extractant d'une fraction inférieure de la colonne de récupération d'extractant et fournir l'extractant à la colonne d'extraction d'aldol.

6. Procédé de préparation de néopentylglycol selon la revendication 4, lequel consiste à récupérer le néopentylglycol d'un flux d'évacuation supérieur de la colonne de purification d'acide hydroxypivalique-ester de néopentylglycol et à renvoyer le néopentylglycol vers une colonne de purification de néopentylglycol.

7. Procédé de préparation de néopentylglycol selon la revendication 1, dans lequel le catalyseur inclut du triéthylamine (TEA).

8. Procédé de préparation de néopentylglycol selon la revendication 1, dans lequel l'extractant inclut du 2-éthylhexanol (2-EH).

9. Procédé de préparation de néopentylglycol selon la revendication 4, dans lequel l'acide hydroxypivalique-ester de néopentylglycol est obtenu d'une partie latérale à une hauteur de 50 à 80 % du haut au bas de la colonne de purification d'acide hydroxypivalique-ester de néopentylglycol.
